(19) Europäisches Patentamt
European Patent Office
Office européen des brevets

(11) EP 4 148 635 A1

(12) EUROPEAN PATENT APPLICATION

(43) Date of publication:
**15.03.2023 Bulletin 2023/11**

(21) Application number: **22194674.2**

(22) Date of filing: **08.09.2022**

(51) International Patent Classification (IPC):
***G06Q 10/04*** *(2012.01)*

(52) Cooperative Patent Classification (CPC):
**G06Q 10/04; G06Q 10/047**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.09.2021 JP 2021147150**

(71) Applicant: **TOYOTA JIDOSHA KABUSHIKI KAISHA**
**Aichi-Ken 471-8571 (JP)**

(72) Inventors:
- **SHIMADA, Masanori**
**Toyota-shi, 471-8571 (JP)**
- **UEDA, Koichi**
**Toyota-shi, 471-8571 (JP)**
- **SUZUKI, Naoto**
**Toyota-shi, 471-8571 (JP)**
- **SAKAYANAGI, Yoshihiro**
**Toyota-shi, 471-8571 (JP)**

(74) Representative: **D Young & Co LLP**
**120 Holborn**
**London EC1N 2DY (GB)**

# (54) CO2 EMISSION CALCULATION DEVICE AND CO2 EMISSION CALCULATION PROGRAM

(57) A $CO_2$ emission calculation device includes a processor configured to calculate a $CO_2$ emission factor of a moving body based on a $CO_2$ emission factor of an energy source supplied to the moving body, and calculate a $CO_2$ emission at a time of a movement of the moving body based on the $CO_2$ emission factor of the moving body.

FIG.1

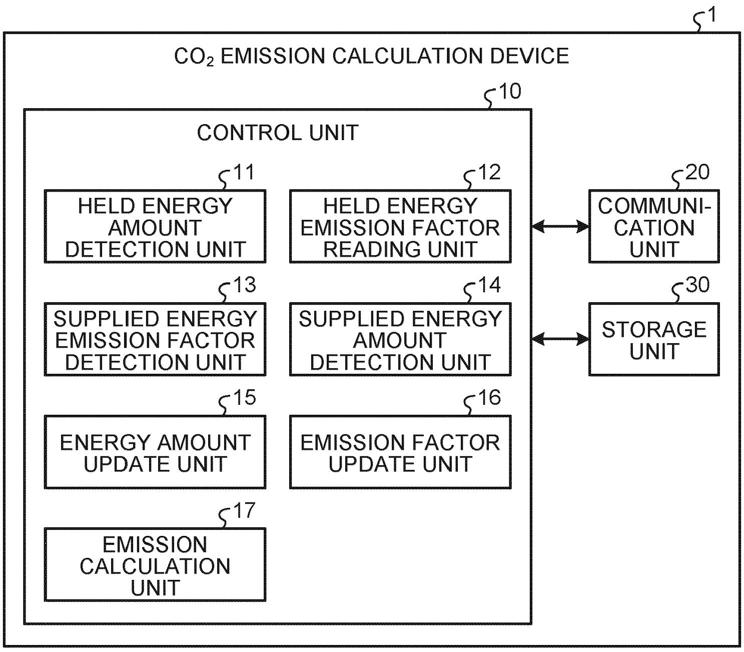


EP 4 148 635 A1

## Description

### BACKGROUND

The present disclosure relates to a $CO_2$ emission calculation device and a computer readable recording medium storing a $CO_2$ emission calculation program.

JP 2010-127690 A discloses a technique of calculating a $CO_2$ emission emitted from an internal combustion of a vehicle, for each section from a departure place to a destination, and searching for a $CO_2$ minimum path through which a total $CO_2$ emission from the departure place to the destination becomes the minimum.

### SUMMARY

There is a need for a technique that enables calculation of a $CO_2$ emission considering a difference in energy resource and "Well to Wheel".

According to one aspect of the present disclosure, there is provided a $CO_2$ emission calculation device including a processor configured to calculate a $CO_2$ emission factor of a moving body based on a $CO_2$ emission factor of an energy source supplied to the moving body, and calculate a $CO_2$ emission at a time of a movement of the moving body based on the $CO_2$ emission factor of the moving body.

### BRIEF DESCRIPTION OF THE DRAWINGS

FIG. 1 is a block diagram illustrating a configuration of a $CO_2$ emission calculation device according to an embodiment; and

FIG. 2 is a flowchart illustrating an example of a processing procedure of a $CO_2$ emission calculation method executed by a $CO_2$ emission calculation device according to the embodiment.

### DETAILED DESCRIPTION

A $CO_2$ emission calculation device and a computer readable recording medium storing a $CO_2$ emission calculation program according to an embodiment of the present disclosure will be described with reference to the drawings. In addition, components in the following embodiments include components that may be easily replaced by the one skilled in the art, or components that are substantially the same.

### $CO_2$ Emission Calculation Device

A $CO_2$ emission calculation device according to an embodiment will be described with reference to FIG. 1. A $CO_2$ emission calculation device is a device for calculating a $CO_2$ emission emitted from a moving body when the moving body moves (runs), considering "Well to Wheel". Note that the "Well to Wheel" is an index indicating an emission degree of $CO_2$ emitted from when oil is extracted from a well (oilfield), until the oil generates a revolving movement of wheels (vehicle wheels) of a vehicle (moving body) through refining and transportation, for example.

Examples of moving bodys to which the $CO_2$ emission calculation device according to an embodiment is applied include a member that is movable by energy obtained from an energy source, such as vehicles, railroads, airplanes, and ships. In addition, vehicles among moving bodys include, for example, an engine vehicle, a Hybrid Electric Vehicle (HEV), a Plug-in Hybrid Electric Vehicle (PHEV), and a Fuel Cell Electric Vehicle (FCEV), a Battery Electric Vehicle (BEV), and the like. In the present embodiment, the description will be given assuming a case where a moving body is a vehicle.

As illustrated in FIG. 1, a $CO_2$ emission calculation device 1 includes a control unit 10, a communication unit 20, and a storage unit 30. Note that the $CO_2$ emission calculation device 1 may be mounted on a moving body such as a vehicle, or may be mounted on a server device or the like that may communicate with a moving body.

Specifically, the control unit 10 includes a processor including a central processing unit (CPU), a digital signal processor (DSP), a field-programmable gate array (FPGA), a graphics processing unit (GPU), and the like, and a memory (main storage unit) including a random access memory (RAM), a read only memory (ROM), and the like.

By executing various programs, the control unit 10 comprehensively controls operations of various components mounted on the $CO_2$ emission calculation device 1. In addition, through the execution of various programs, the control unit 10 functions as a held energy amount detection unit 11, a held energy emission factor reading unit 12, a supplied energy emission factor detection unit 13, a supplied energy amount detection unit 14, an energy amount update unit 15, an emission factor update unit 16, and an emission calculation unit 17.

The held energy amount detection unit 11 detects an energy amount (V_base) of an energy source held by a moving body. In the present embodiment, an energy amount of an energy source held by a moving body is defined as a "held energy amount".

Here, an "energy source" refers to a generation source of energy for operating a moving body, and examples include liquid fuel such as gasoline and light oil, electrical power, hydrogen, liquefied petroleum gas, gas fuel such as natural gas, and the like. In addition, among energy sources, while some energy sources emit $CO_2$ when generating energy, such as thermal electric generation, for example, other energy sources do not emit $CO_2$ when generating energy, such as solar energy generation.

For example, in a case where an energy source held by a moving body is liquid fuel, the held energy amount detection unit 11 may detect, as a held energy amount, a fuel amount [L] corresponding to an indicated value of a fluid level sensor or the like that is mounted on the moving body.

In addition, in a case where an energy source held by a moving body is electrical power, the held energy amount detection unit 11 may detect, as a held energy amount, electrical energy [kWL] corresponding to an indicated value of a voltage sensor or the like that is mounted on the moving body.

In addition, in a case where an energy source held by a moving body is gas fuel, the held energy amount detection unit 11 may detect, as a held energy amount, a fuel amount [kg] corresponding to an indicated value of a pressure sensor or the like that is mounted on the moving body. Note that the held energy amount detection unit 11 may store held energy amounts detected using these methods, into the storage unit 30.

The held energy emission factor reading unit 12 reads out a $CO_2$ emission factor (K_base) of an energy source held by a moving body. In the present embodiment, a $CO_2$ emission factor of an energy source held by a moving body is defined as a "held energy emission factor". Note that the held energy emission factor refers to a $CO_2$ emission factor of an energy source already held by a moving body when an energy source is supplied to the moving body from external supply equipment or the like.

The held energy emission factor reading unit 12 reads out, as a held energy emission factor, a previous value of a $CO_2$ emission factor per unit energy that has been calculated and updated by the emission factor update unit 16 to be described later, for example, and stored in the storage unit 30.

Note that the held energy emission factor is calculated by the emission factor update unit 16 to be described later, each time a moving body performs resupply of an energy source such as fueling and charging, and is accordingly updated to a latest value. Thus, the held energy emission factor is not a fixed value, and varies depending on the timing at which the held energy emission factor reading unit 12 reads out the held energy emission factor. In addition, a value of the held energy emission factor becomes lower in a case where the energy source is generated using natural energy (for example, sunlight, wind power, tidal power, geothermal heat, etc.), and a value of the held energy emission factor becomes higher in other cases, for example.

Here, in a case where a $CO_2$ emission factor has not been calculated by the emission factor update unit 16 yet (in a case where a previous value of a $CO_2$ emission factor does not exist), for example, a $CO_2$ emission factor of general "Tank To Wheel" may be used as a held energy emission factor. Note that the "Tank To Wheel" is an index indicating an emission degree of $CO_2$ emitted from a state in which fuel is already stored in a fuel tank, until the fuel generates a revolving movement of wheels (vehicle wheels) of a vehicle, for example.

The supplied energy emission factor detection unit 13 detects a $CO_2$ emission factor (K_add) of an energy source supplied to a moving body. In the present embodiment, a $CO_2$ emission factor of an energy source supplied to a moving body is defined as a "supplied energy emission factor". The supplied energy emission factor is predefined for each supply equipment (for example, gas station, charging station, hydrogen filling station, etc.) or business operator (for example, power company, oil distributor, etc.) that supplies an energy source.

The supplied energy emission factor detection unit 13 detects a supplied energy emission factor by acquiring a $CO_2$ emission factor from a server device or the like that is provided in supply equipment or a business operator (hereinafter, referred to as "supply equipment or the like"), via a wired or wireless network, for example.

In addition, for example, in a case where supply equipment or the like is a gas station, the supplied energy emission factor detection unit 13 may detect a supplied energy emission factor by reading out a $CO_2$ emission factor per liter that is described on a fueling nozzle of gasoline, using a camera, a sensor, or the like. Note that, in a case where supply equipment or the like is a gas station, in a case where a $CO_2$ emission factor may not be acquired from a fueling nozzle of gasoline, the supplied energy emission factor detection unit 13 may identify the position of the gas station from GPS information or the like of the moving body, and acquire a $CO_2$ emission factor per liter of the identified gas station, from a database or the like on a network.

Here, an energy source is sometimes supplied from equipment or energy other than supply equipment or the like. For example, in a case where a moving body is a BEV, electrical power generated by regeneration at the time of deceleration sometimes serves as an energy source. In addition, in some cases, a moving body is provided with a solar panel or the like, and electrical power generated by sunlight (natural energy) serves as an energy source. In this manner, in a case where an energy source obtained by a moving body itself or natural energy is used, the supplied energy emission factor detection unit 13 sets 0 as a supplied energy emission factor of the energy source.

The supplied energy amount detection unit 14 detects an energy amount (V_add) of an energy source supplied to a moving body. In the present embodiment, an energy amount of an energy source supplied to a moving body is defined as a "supplied energy amount".

The supplied energy amount detection unit 14 detects a supplied energy amount by acquiring a supplied amount of an energy source (for example, a supplied amount or the like of gasoline) from a server device or the like that is provided in supply equipment or the like, via a wired or wireless network, for example. In addition, the supplied energy amount detection unit 14 may detect an increase in energy source using a sensor or the like (for example, a fluid level sensor or the like) mounted on a moving body, when an energy source is supplied from supply equipment or the like, for example, and regard the increase as a supplied energy amount.

The energy amount update unit 15 calculates an energy amount (V_base') of a moving body based on a held energy amount and a supplied energy amount, and updates a previously-calculated energy amount. Here, an "energy amount of a moving body" refers to an energy amount of the entire moving body that considers an energy amount of an energy source (i.e., held energy amount (V_base)) already held by the moving body before an energy source is supplied from supply equipment or the like, and an energy amount of an energy source (i.e., supplied energy amount V_add)) supplied from supply equipment or the like to the moving body. The energy amount update unit 15 calculates an energy amount (V_base') of a moving body by the following formula (1), for example. Then, a previous value of an energy amount that is stored in the storage unit 30 is updated to a value calculated this time.

$$V_base' = V_base + V_add \qquad (1)$$

The emission factor update unit 16 calculates a $CO_2$ emission factor (K_base') of a moving body based on a held energy amount, a supplied energy amount, a held energy emission factor, and a supplied energy emission factor, and updates a previously-calculated $CO_2$ emission factor. Here, a "$CO_2$ emission factor of a moving body" refers to a $CO_2$ emission factor of the entire moving body that considers a held energy emission factor and a supplied energy emission factor. The emission factor update unit 16 calculates a $CO_2$ emission factor (K_base') of a moving body by the following formula (2), for example. Then, a previous value of a $CO_2$ emission factor that is stored in the storage unit 30 is updated to a value calculated this time.

$$K_base' = (V_base \times K_base + V_add \times K_add)/(V_base + V_add) \qquad (2)$$

The emission calculation unit 17 calculates a $CO_2$ emission at the time of the movement of a moving body based on a $CO_2$ emission factor of the moving body that has been calculated by the emission factor update unit 16.

Here, the emission factor update unit 16 calculates a $CO_2$ emission factor of a moving body after setting a $CO_2$ emission factor of an energy source generated using natural energy, to a smaller $CO_2$ emission factor than a $CO_2$ emission factor of an energy source generated not using natural energy.

For example, as described above, in a case where electrical power is obtained by the regeneration of a moving body, or in a case where electrical power is obtained using natural energy, the supplied energy emission factor detection unit 13 calculates a $CO_2$ emission factor of the moving body by setting a supplied energy emission factor of the electrical power to 0.

In addition, for example, in a case where a moving body is a vehicle (for example, series HEV) including an engine and an electric generator, a $CO_2$ emission factor of the moving body may be calculated by calculating fuel amount $\times$ $CO_2$ emission factor at the stage of fuel consumption caused by electric generation, and setting a $CO_2$ emission factor on a side of electrical power that is accordingly charged, to 0.

In addition, for example, in a case where a moving body is a PHEV including a plurality of energy supply means, a $CO_2$ emission factor of the moving body may be calculated after identifying the respective capacities of fuel and a battery.

The emission calculation unit 17 calculates a $CO_2$ emission at the time of the movement of a moving body. The emission calculation unit 17 calculates a $CO_2$ emission per unit time or unit distance by multiplying a change amount of a consumption of an energy source of a moving body (for example, consumption of fuel, consumption of electrical power, or the like) by the $CO_2$ emission factor calculated by the emission factor update unit 16, for example. Then, by integrating $CO_2$ emissions per unit time or unit distance, a $CO_2$ emission at the time of the movement of a moving body is calculated.

The communication unit 20 includes, for example, a local area network (LAN) interface board, a radio communication circuit for radio communication, and the like. The communication unit 20 communicates with a server device or the like that is provided in supply equipment or the like by connecting to a wired or wireless network when the supplied energy emission factor detection unit 13 detects a supplied energy emission factor or when the supplied energy amount detection unit 14 detects a supplied energy amount, for example.

The storage unit 30 includes an erasable programmable ROM (EPROM), a hard disk drive (HDD), removable media, and the like. Examples of removable media include a universal serial bus (USB) memory and disc recording media such as a compact disc (CD), a digital versatile disc (DVD), and a Blu-ray (registered trademark) disc (BD). The storage unit 30 may store an operating system (OS), various programs, various tables, various databases, and the like.

A held energy amount detected by the held energy amount detection unit 11, a held energy emission factor read out by the held energy emission factor reading unit 12, a supplied energy $CO_2$ emission factor detected by the supplied energy emission factor detection unit 13, a supplied energy amount detected by the supplied energy amount detection unit 14, an energy amount calculated and updated by the energy amount update unit 15, a $CO_2$ emission factor calculated and updated by the emission factor update unit 16, a $CO_2$ emission calculated by the emission calculation unit 17, and the like are stored into the storage unit 30 as necessary.

$CO_2$ Emission Calculation Method

An example of a processing procedure of a $CO_2$ emission calculation method executed by the $CO_2$ emission calculation device 1 according to an embodiment will be described with reference to FIG. 2. Note that, among processes in Steps S1 to S7 illustrated in the drawing, an order of the processes in Steps S1 to S4 is not specifically limited. In other words, the processes in Steps S1 to S4 may be executed in any order.

First of all, the held energy amount detection unit 11 detects a held energy amount of a moving body (Step S1). Subsequently, the held energy emission factor reading unit 12 reads out a held energy $CO_2$ emission factor of the moving body (Step S2). Subsequently, the supplied energy emission factor detection unit 13 detects a supplied energy $CO_2$ emission factor of the moving body (Step S3). Subsequently, the supplied energy amount detection unit 14 detects a supplied energy amount of the moving body (Step S4) .

Subsequently, the energy amount update unit 15 calculates an energy amount of the moving body using the above-described formula (1), and updates a previously-calculated energy amount (Step S5). Subsequently, the emission factor update unit 16 calculates a $CO_2$ emission factor of the moving body using the held energy amount, the supplied energy amount, and the above-described formula (2), and updates a previously-calculated $CO_2$ emission factor (Step S6). Subsequently, the emission calculation unit 17 calculates a $CO_2$ emission at the time of the movement of the moving body based on the $CO_2$ emission factor of the moving body that has been calculated by the emission factor update unit 16 (Step S7). As described above, the processing of the $CO_2$ emission calculation method is completed.

According to the $CO_2$ emission calculation device and the $CO_2$ emission calculation program according to the above-described embodiment, a $CO_2$ emission emitted from a moving body may be calculated considering a difference in energy resource and "Well to Wheel".

More specifically, in technologies proposed so far, calculation accuracy of a $CO_2$ emission has been insufficient because a difference between general light oil and biodiesel, or a difference between electricity generated by thermal electric generation and electricity generated by solar electric generation, in BEV running, or a difference among grey hydrogen, green hydrogen, and blue hydrogen may not be discriminated, for example. On the other hand, according to the $CO_2$ emission calculation device and the $CO_2$ emission calculation program according to an embodiment, a $CO_2$ emission in total may be calculated including a generation process of an energy source, considering a difference in energy resource and "Well to Wheel".

According to the present disclosure, a $CO_2$ emission emitted from a moving body may be calculated considering a difference in energy resource and "Well to Wheel".

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

**1.** A $CO_2$ emission calculation device comprising
a processor configured to:

calculate a $CO_2$ emission factor of a moving body based on a $CO_2$ emission factor of an energy source supplied to the moving body; and
calculate a $CO_2$ emission at a time of a movement of the moving body based on the $CO_2$ emission factor of the moving body.

**2.** The $CO_2$ emission calculation device according to claim 1, wherein the processor is configured to calculate the $CO_2$ emission factor of the moving body based on, in addition to the $CO_2$ emission factor of an energy source supplied to the moving body, a $CO_2$ emission factor of an energy source already held by the moving body when the energy source is supplied.

**3.** The $CO_2$ emission calculation device according to claim 1 or 2, wherein the processor is configured to set a $CO_2$ emission factor of an energy source generated using natural energy, to a smaller $CO_2$ emission factor than a $CO_2$ emission factor of an energy source generated not using the natural energy.

**4.** A $CO_2$ emission calculation program for causing a processor to execute:

calculating a $CO_2$ emission factor of a moving body based on a $CO_2$ emission factor of an energy source supplied to the moving body; and
calculating a $CO_2$ emission at a time of a movement of the moving body based on the $CO_2$ emission factor of the moving body.

**5.** The $CO_2$ emission calculation program according to claim 4, wherein the program causes the processor to execute: calculating the $CO_2$ emission factor of the moving body based on, in addition to the $CO_2$ emission factor of an energy source supplied to the moving body, a $CO_2$ emission factor of an energy source already held by the moving body when the energy source is supplied.

**6.** The $CO_2$ emission calculation program according to claim 4 or 5, wherein the program causes the processor to execute:
setting a $CO_2$ emission factor of an energy source generated using natural energy, to a smaller $CO_2$ emission factor than a $CO_2$ emission factor of an energy source generated not using the natural energy.

FIG.1

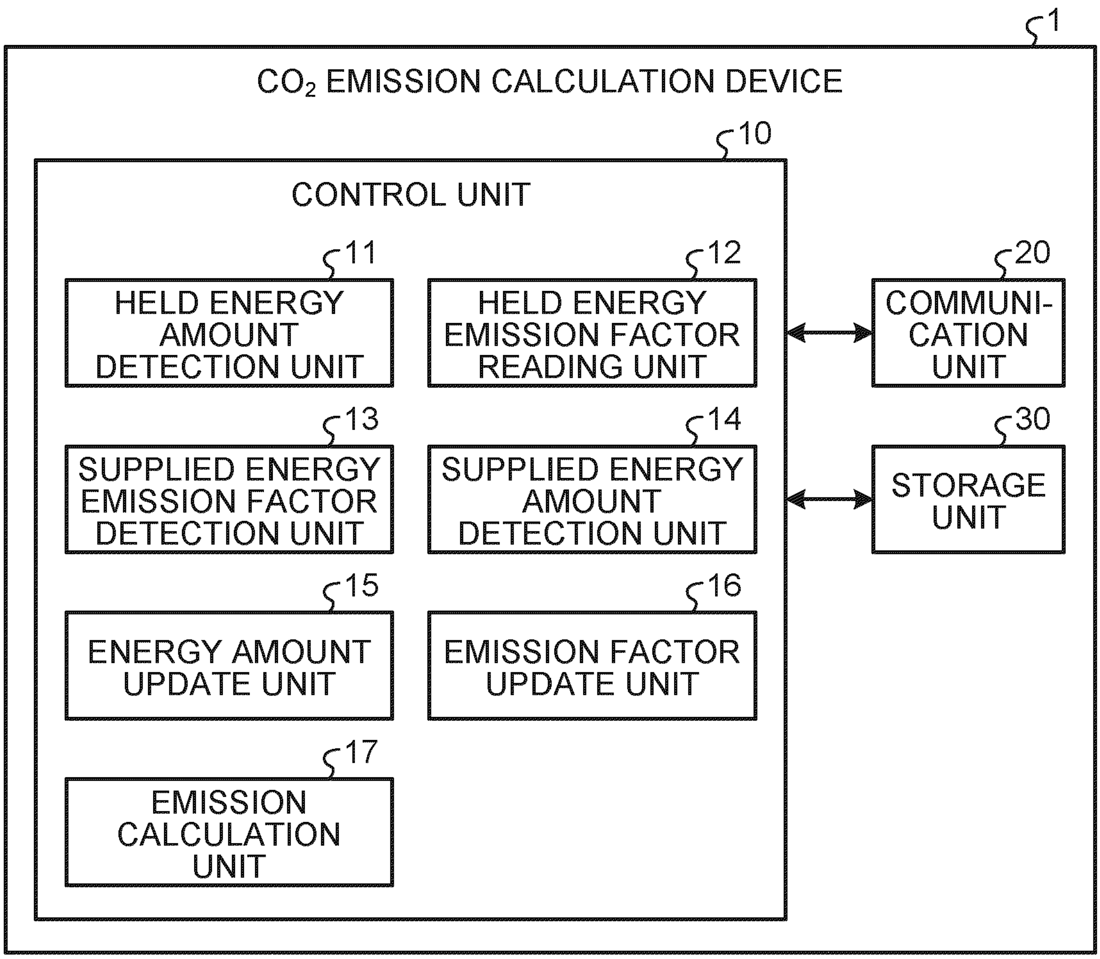
1
CO2 EMISSION CALCULATION DEVICE
10
CONTROL UNIT
11
HELD ENERGY AMOUNT DETECTION UNIT
12
HELD ENERGY EMISSION FACTOR READING UNIT
13
SUPPLIED ENERGY EMISSION FACTOR DETECTION UNIT
14
SUPPLIED ENERGY AMOUNT DETECTION UNIT
15
ENERGY AMOUNT UPDATE UNIT
16
EMISSION FACTOR UPDATE UNIT
17
EMISSION CALCULATION UNIT
20
COMMUNI-CATION UNIT
30
STORAGE UNIT

FIG.2

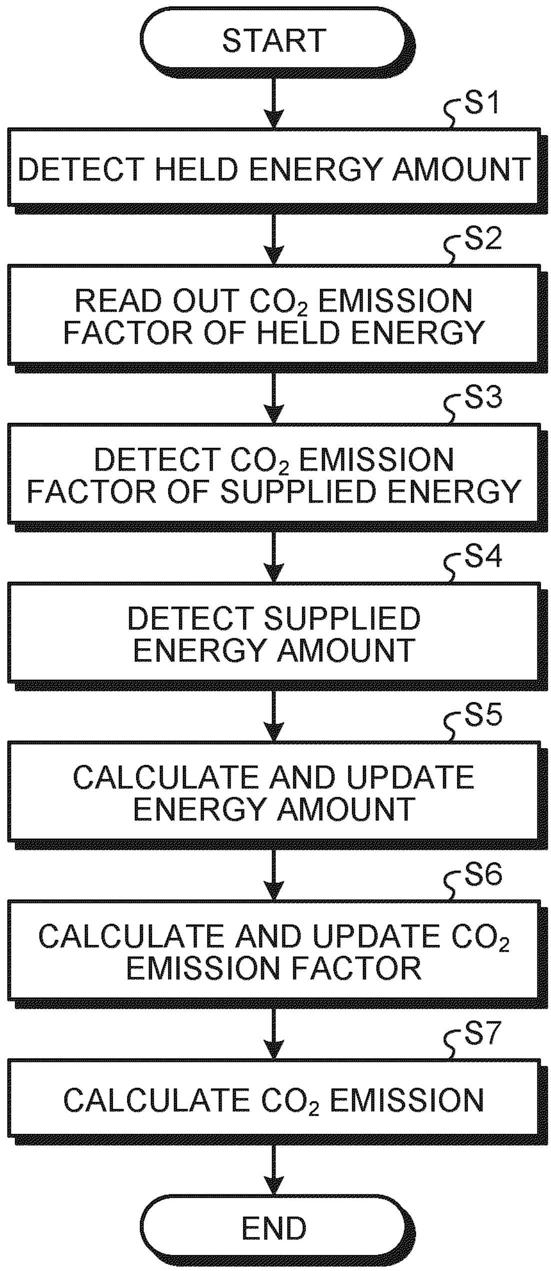
START
S1
DETECT HELD ENERGY AMOUNT
S2
READ OUT $CO_2$ EMISSION FACTOR OF HELD ENERGY
S3
DETECT $CO_2$ EMISSION FACTOR OF SUPPLIED ENERGY
S4
DETECT SUPPLIED ENERGY AMOUNT
S5
CALCULATE AND UPDATE ENERGY AMOUNT
S6
CALCULATE AND UPDATE $CO_2$ EMISSION FACTOR
S7
CALCULATE $CO_2$ EMISSION
END

Europäisches Patentamt
European Patent Office
Office européen des brevets

# EUROPEAN SEARCH REPORT

Application Number
EP 22 19 4674

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/232027 A1 (REICH DANIEL [US] ET AL) 5 September 2013 (2013-09-05) * paragraphs [0027] - [0028], [0040] - [0049]; figures 1-10; tables 1-3 * ----- | 1-6 | INV. G06Q10/04 |

TECHNICAL FIELDS SEARCHED (IPC)

G06Q

The present search report has been drawn up for all claims

1

| Place of search | Date of completion of the search | Examiner |
|---|---|---|
| Munich | 23 December 2022 | Bîrlescu, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 19 4674

This annex lists the patent family members relating to the patent documents cited in the above-mentioned European search report. The members are as contained in the European Patent Office EDP file on
The European Patent Office is in no way liable for these particulars which are merely given for the purpose of information.

23-12-2022

| Patent document cited in search report | Publication date | Patent family member(s) | Publication date |
|---|---|---|---|
| US 2013232027 A1 | 05-09-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010127690 A